# EUROPÄISCHE PATENTSCHRIFT

(11) **EP 1 438 933 B1**
(45) Veröffentlichungstag und Bekanntmachung des Hinweises auf die Patenterteilung: **06.04.2005**
(21) Anmeldenummer: 03016156.6
(22) Anmeldetag: 16.07.2003
(51) Int. Cl.: A61F 2/36

(54) **Hüftprothese mit einem in den Markkanal des Oberschenkelknochens zu verankernden Schaft**
Hip prosthesis with a stem to be implanted into the medullary canal of the femur
Prothese de hanche avec un tige pour être implanté dans le canal médullaire du femur

(30) Priorität: 17.01.2003 EP 03001040
(43) Veröffentlichungstag der Anmeldung: 21.07.2004
(73) Patentinhaber: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll & Partner

(56) Entgegenhaltungen:
- EP-A- 0 677 281
- EP-A- 0 682 924
- EP-A- 0 845 251
- WO-A-02/100302
- FR-A- 2 686 789
- US-A- 5 156 627
- US-A- 5 480 452

## Beschreibung

Die Diaphyse des Oberschenkelknochens, d.h. der gestreckte Teil dieses Knochens, der beim kleinen Trochanter beginnt, hat eine verhältnismäßig dicke und kräftige Knochenrinde (Corticalis) und ist deshalb zur Verankerung eines Prothesenschafts besonders geeignet. Es sind Prothesen bekannt, die dies dadurch nutzen, daß ihr in die Diaphyse des Oberschenkelknochens hineinragender Schaft einen Schaftquerschnitt aufweist, der der Größe des Markkanals angepaßt ist und einen etwa konstanten Querschnitt (US-A-4,549,319, DE-A-2839092) hat. Es gibt auch Prothesen, deren Schaftquerschnitt sich im distalen Abschnitt zum Ende hin verjüngt (EP-A-135755, US-A-2,719,522, US-A-3,067,740). Dabei kann es sich um solche handeln, die mit Zement zu verankern sind und auf deren Querschnittunterschied gegenüber dem Markkanal es daher nicht ankommt (Schneider: Die Totalprothese der Hüfte, S. 120; FR-A-2791252; GB-A-2203943) oder bei denen der Schaft sich aufgrund seiner Keilform in der Markhöhle verkeilen soll (Müller, a.a.O., S. 214 ff.; US-A-5156627). Letztere haben den Nachteil, daß die Verkeilung zu hoher Kraftkonzentration an den Verkeilungsstellen führt. Dies ist insbesondere dann der Fall, wenn sie zwecks guter Verbindung mit dem Knochen und zur Verbesserung der Verdrehsicherheit in der lateralen Fläche oder an deren Kanten mit vom Schaftkern vorstehenden Längsrippen versehen sind (EP-A-682924, EP-A-677281, US-A-5593446, DE-U-29907259, US-4784124, EP-845251, EP-A-821923). Deshalb zieht man es im allgemeinen vor, solche Rippen an der ventralen oder dorsalen Seite anzubringen (EP-A-238860, EP-A-159462, WO-02100302). Jedoch gewährleistet auch eine rippenlose laterale Seite keine großflächige Anlage an der Knochenrinde, weil diese von zufälligen Formübereinstimmungen zwischen Prothesenschaft und Innenfläche der Knochenrinde abhängig ist. Erwünscht ist aber eine großflächige, zementfreie Kraftübertragung, die aber nach bisheriger Kenntnis eine individuelle Anpassung des Prothesenschafts an die Form des Markkanals erforderlich macht, die wegen des sehr hohen Aufwands normalerweise nicht in Betracht kommt. Auch haben sie eine unzureichende Sicherheit gegen Verdrehung.

Der Erfindung liegt daher die Aufgabe zugrunde, eine Hüftprothese zu schaffen, deren Schaft bei zementfreier Implantation zu großflächiger Kraftübertragung im diaphysären Bereich bei guter Verdrehsicherheit in der Lage ist.

Ausgehend von solchen bekannten Prothesen, deren sich zum distalen Ende hin verjüngender Schaft zumindest auf der lateralen und der medialen Seite Längsrippen aufweist (EP-A-677281), besteht die erfindungsgemäße Lösung in den Merkmalen des Anspruchs 1 und vorzugsweise denjenigen der Unteransprüche.

Demgemäß ist vorgesehen, daß der Kernquerschnitt des Schafts in 1 cm Abstand vom distalen Ende im wesentlichen rechteckig begrenzt ist mit einem Achsenverhältnis von mindestens 1,5, daß er mindestens nahe seinem distalen Ende an seinen beiden lateralen Kanten je eine Rippe trägt und daß die Höhe der Längsrippen von proximal nach distal anwächst. Die gewünschte, großflächige Kraftübertragung kommt dadurch zustande, daß die von dem Schaftkern gebildeten, keilförmig anwachsenden Flächen zwischen den Rippen beim Eintreiben des Schafts in den Markkanal zu einer Verdichtung der dort vorhandenen lamellaren Knochensubstanz führen, die dabei zwischen den Rippen festgehalten wird. Dabei wird der Schaft beim Einführen durch die Rippen geführt, so daß er nicht seitlich ausweichen kann, und werden durch das Zusammenschieben und Komprimieren von Knochensubstanz zwischen den Rippen zusätzliche Kraftübertragungsbereiche geschaffen. Auch wenn die Menge des zu verpressenden Knochenmaterials und seine örtliche Anordnung von Fall zu Fall unterschiedlich sein mag, wird dadurch in jedem Fall bereichsweise eine gute Kraftübertragung vom Schaft über die verpreßte Knochensubstanz zur harten Knochenrinde hin geschaffen. Dies ergibt nicht nur eine gute Anfangsfestigkeit, sondern auch die Möglichkeit späteren Einwachsens neuer Knochensubstanz in die verbliebenen Zwischenräume und damit gute Voraussetzungen für einen langfristig guten Prothesensitz.

Dies schließt nicht aus, daß die Prothese auch in ihrem proximalen Bereich zur Kraftübertragung eingerichtet ist. Die erfindungsgemäße distale Formgebung ist sogar besonders geeignet für solche Fälle, in denen die Prothese auch proximale Kraftübertragungseinrichtungen aufweist.

Damit die von dem konischen Schaftkern komprimierte Knochensubstanz zwischen den Rippen gut gesichert wird, sollten an den nach lateral bzw. medial gewendeten Oberflächenabschnitten des Kerns mindestens je drei Rippen vorhanden sein. Zusätzlich sind zweckmäßigerweise auch auf der vorderen und rückseitigen Seite Längsrippen vorhanden.

Für eine effektive Kompression der Knochensubstanz hat sich eine Verjüngung des Kernquerschnitts zu seinem Ende hin bewährt, die über eine Länge von mindestens 4 cm durchschnittlich mindestens 8 mm²/cm Länge beträgt und vorzugsweise über 10 mm²/cm liegt. Sie sollte 20 mm²/cm nicht überschreiten und vorzugsweise unter 16 mm²/cm im Mittel liegen.

In der LM-Ebene sollte die Verjüngung der Kernabmessung über 4 cm Länge mindestens 0,5 mm/cm und vorzugsweise etwa 1 mm/cm betragen. Die Rippenhöhe braucht nicht groß zu sein. Sie sollte im Mittel unter 2 mm liegen. Im allgemeinen genügt eine mittlere Rippenhöhe von 1 mm und weniger. Zweckmäßig ist eine Schaftausbildung, bei der die Rippenhöhe vom proximalen Ende des distalen Abschnitts bis zu dessen distalen Ende von 0 auf 0,5 bis 1,5 mm, vorzugsweise etwa 1 mm, anwächst.

Da hauptsächlich der Kernquerschnitt für die Kompression der Knochensubstanz verantwortlich ist und die Rippen lediglich eine Halte- und Führungsfunktion haben, sollte deren Querschnittsausdehnung gering bleiben. Ihre mittlere Erstreckung ihres Querschnitts in Umfangsrichtung sollte nicht größer als 30 % ihres Mittenabstands sein und bspw. in der Größenordnung von 20 % liegen. Dabei ist für die Bestimmung ihres Querschnitts ihre Höhe zwischen der Oberfläche des Kernquerschnitts und ihrer Spitze zugrunde zu legen. Es soll in dem gerippten Teil des Schafts außer den Rippen keine weiteren Vorsprünge geben. Falls doch irgendwelche zusätzlichen Vorsprünge vorhanden sind, sollen sie nicht weiter als die Rippen vorragen.

Die Länge des gerippten, distalen Schaftabschnitts liegt zweckmäßigerweise bei mindestens 4 cm und vorzugsweise bei etwa 5 bis 8 cm. Dabei wird vorausgesetzt, daß der proximale Beginn des distalen Schaftabschnitts etwa 7 bis 9 cm unterhalb der Höhe des Gelenkkopfs liegt.

Die Flanken der Rippen schließen in dem Bereich, wo sie ihre größte Höhe haben, mit dem Radius durch die Rippenmitte einen Winkel von vorzugsweise nicht mehr als 30° ein. Weiter vorzugsweise ist dieser Winkel kleiner als 20°. Eine Ausnahme von dieser Regel kann dann gelten, wenn der Schaft bei der Herstellung quer zu seiner Längsrichtung entformt werden muß. Gegenüber der Entformungsrichtung - dies ist im allgemeinen die Richtung quer zu der Ebene, in welcher die Haupterstreckung der Prothese liegt - sollen die Rippen in diesem Fall nicht hinterschnitten sein.

Im oberen und mittleren Bereich, d. h. auch am proximalen Ende des distalen, gerippten Schaftabschnitts, hat der Schaft einen langgestreckten Querschnitt, dessen längere Achse in der lateral-medialen Ebene liegt. Das Achsenverhältnis am proximalen Ende des distalen Abschnitts sollte mindestens 1,2 betragen. Dieser Querschnitt kann oval sein. Jedoch ist es zweckmäßig, daß mindestens der gerippte Abschnitt des Schafts im Querschnitt langgestreckt rechteckig mit der längeren Achse in LM-Richtung ausgebildet ist, wobei das Achsenverhältnis mindestens 1,4 beträgt. Am distalen Ende, an dem der Querschnitt mit Rücksicht auf eine möglicherweise vorhandene Endabrundung in 1 cm Abstand vom Ende gemessen wird, ist das Achsenverhältnis mindestens 1,5. Dabei ist mindestens an den beiden lateralen Kanten je eine Rippe vorgesehen, da an dieser Stelle die Führungsaufgabe, die den Rippen zugeteilt ist, besonders wichtig ist. Damit nicht eine dieser Kanten am Führungskontakt mit der Begrenzung des Markkanals durch weiter vorspringende Teile des Schafts gehindert wird, soll die Begrenzung des Schaftkernquerschnitts zwischen den beiden an den lateralen Kanten befindlichen Rippen nicht weiter lateral vorragen als diese. Falls sich zwischen den beiden an den lateralen Kanten befindlichen Rippen noch eine weitere laterale Rippe befindet, soll diese nicht mehr als 2 mm, vorzugsweise nicht mehr als 1 mm, weiter nach lateral vorragen als diese.

Auch an den medialen Kanten kann je eine Rippe vorgesehen sein. Dabei ist es zweckmäßig, wenn an den ventralen und dorsalen Seiten des Schaftquerschnitts der Schaftkernquerschnitt zwischen den Rippen, die den diese Seite begrenzenden lateralen und medialen Kanten zugeordnet sind, nicht mehr als 1 mm nach ventral bzw. dorsal über diese hinaus vorragen. Wenn an dieser Seite zwischen den Kanten noch eine oder mehrere Rippe(n) angeordnet sind, sollen diese nicht mehr als 2 mm, vorzugsweise nicht mehr als 1 mm nach ventral bzw. dorsal vorragen als die an den Kanten vorgesehenen Rippen.

Damit die Rippen ihre Führungsaufgabe leichter erfüllen können, sollen sie nach einem weiteren Merkmal der Erfindung rauh ausgebildet sein, um in die harte Knochenrinde leichter einschneiden zu können. Um eine unerwünscht starke Verletzung des Knochens zu vermeiden, soll diese Rauhigkeit nicht zu grob sein. Der Mittenabstand benachbarter Körner oder Zacken soll 0,5 mm nicht überschreiten und liegt im Mittel zweckmäßigerweise in der Größenordnung von 0,1 mm.

Die Erfindung wird im folgenden näher unter Bezugnahme auf die Zeichnung erläutert, die ein vorteilhaftes Ausführungsbeispiel der Prothese darstellt. Es zeigen:
- Fig. 1: eine Ansicht von vorne,
- Fig. 2: eine Ansicht von medial,
- Fig. 3: eine Ansicht von oben,
- Fig. 4 bis 6: Schnitte durch den Schaft in verschiedenen Höhen desselben, und
- Fig. 7: einen Schnitt entsprechend Fig. 5 mit rechteckigem Schaftkernquerschnitt.

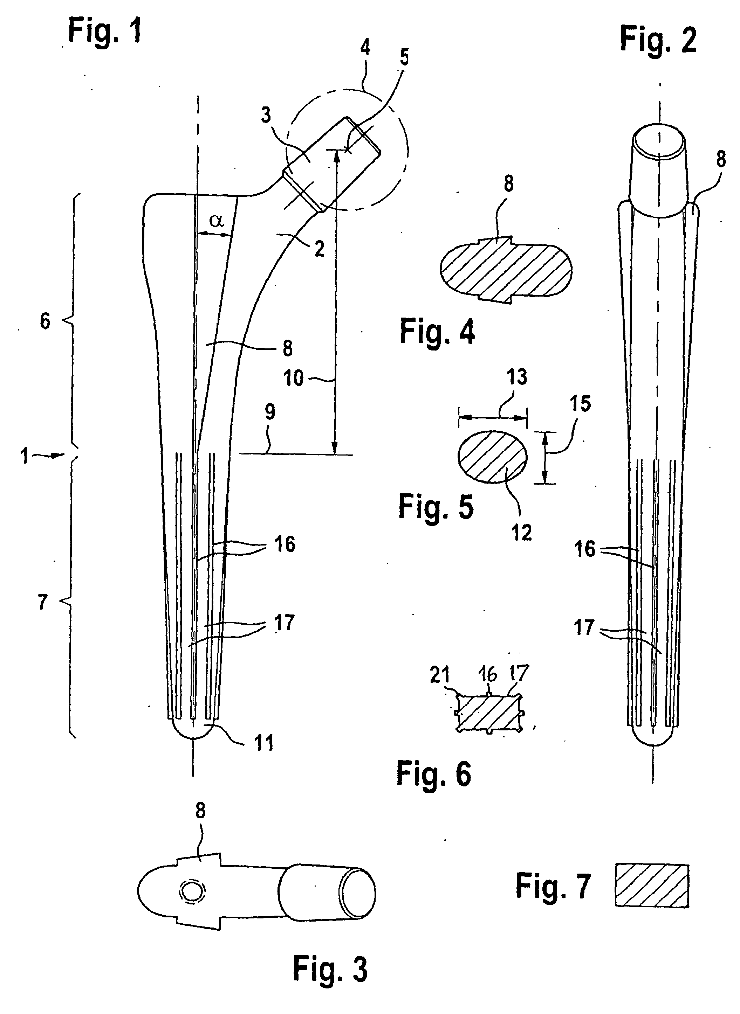

Die Prothese besteht aus einem Schaft 1, einem Hals 2 und einem Konus 3 zum Aufsetzen eines Gelenkkopfs 4, dessen Umfang strichpunktiert angedeutet ist und der einen Mittelpunkt 5 aufweist. Es handelt sich um eine sogenannte Geradschaftprothese. Bei diesem bekannten Prothesentyp ist der Schaft in seiner Gesamtheit im wesentlichen gerade ausgebildet. Er wird im Gegensatz zu Prothesen, deren Schaft im proximalen Bereich so gekrümmt ist, daß ihre Richtung sich der Richtung des Kopfhalses 2 angleicht, in nur einer Richtung in die im Knochen zu seiner Aufnahme geschaffene Höhlung eingeführt. Der Schaft setzt sich zusammen aus einem proximalen Abschnitt 6 und einem distalen Abschnitt 7. Der proximale Abschnitt kann mit besonderen Einrichtungen zur besseren Kraftübertragung auf die im epiphysären Bereich des Oberschenkelknochens den Schaft umgebenden Knochensubstanz ausgerüstet sein. Im konkreten Fall dient dafür ein Rippenpaar 8. Der Schaftquerschnitt ist in diesem Bereich langgestreckt in LM-Richtung, wie Fig. 4 und 5 bzw. 7 zeigen.

Die Übergangsstelle 9 zwischen dem proximalen und dem distalen Abschnitt des Schafts ist so angeordnet, daß sie im implantierten Zustand wenig unterhalb des kleinen Trochanters zu liegen kommt und der distale Schaftabschnitt 7 demzufolge in einem Bereich des Markkanals liegt, in welchem dieser durch eine starke Knochenrinde begrenzt ist, von der aus sich einige lamellare Knochensubstanz in den Markkanal hinein erstreckt. Die Übergangsstelle braucht an der Prothese nicht besonders markiert zu sein. Man kann sie dadurch erkennen, daß sie dort liegt, wo sich im implantierten Zustand etwa der kleine Trochanter oder besser dessen Unterkante befindet. Sie liegt etwas 7 bis 9 cm tiefer als der Mittelpunkt 5 des Gelenkkopfes, gemessen gemäß Pfeil 10 in Schaftrichtung.

Der distale Schaftabschnitt 7 hat unterhalb der Übergangsstelle 9 eine Länge von etwa 4 bis 8, vorzugsweise etwa 6 bis 7 cm. Sein Kern 12 verjüngt sich von seinem proximalen Ende 9 bis zu seinem distalen Ende 11 hin in einem Verhältnis von etwa 10 bis 15 mm²/cm. Die Verjüngung findet hauptsächlich auf den lateralen und medialen Seiten statt. Die LM-Abmessung 13 am oberen Ende 9 des distalen Abschnitts, die etwa 17 mm beträgt, vermindert sich bis zum distalen Ende 11 gemäß Fig. 6 auf etwa 14 mm.

Die Oberfläche des Schaftkerns ist im distalen Abschnitt mit Rippen 16 besetzt, die zwischen sich Flächenstreifen 17 der Schaftkernoberfläche einschließen. Die an den Längskanten des Rechteckquerschnitts angeordneten Rippen sind in Fig. 6 mit der Bezugsziffer 21 bezeichnet. Die Rippen 16, 21 gehen am Übergang 9 mit der Höhe Null in die Schaftoberfläche über und erreichen am distalen Ende 11 eine Höhe von etwa 1 mm über der Schaftkernoberfläche. Die zwischen den Rippen gebildeten Flächenstreifen 17 wirken infolge der Querschnittsverringerung des Schaftkerns von proximal nach distal als Keilflächen, die beim Eintreiben des Schafts in den Markkanal die dort befindliche, vornehmlich lamellare Knochensubstanz im Zwischenraum zwischen der Oberfläche des Schaftkerns und der kortikalen Markraumbegrenzung komprimieren. Sie kann dabei nicht seitlich weggequetscht werden und ausweichen, weil sie durch die Rippen 16 festgehalten wird. Dadurch werden verdichtete, kompakte Kraftübertragungsbrücken zwischen dem Prothesenschaft und der kortikalen Markraumbegrenzung auch in solchen Bereichen des Schaftquerschnitts geschaffen, die ohne diese Kompression nicht an die kortikale Begrenzung des Markraums heranreichen würden und sich deshalb an der Kraftübertragung nicht beteiligen könnten. Da die Abnahme der Schaftquerschnittsabmessung in LM-Richtung stärker ist als in AP-Richtung, findet die stärkste Kompression an den lateralen und medialen Flanken des Schafts statt. Dort werden daher auch die wirksamsten Kraftübertragungsbrücken durch Kompression von Knochensubstanz entstehen. Dies ist deshalb vorteilhaft, weil der größte Teil der Kräfte in dieser Richtung zwischen Prothesenschaft und Knochen zu übertragen ist. Aber auch an den anterioren und posterioren Seiten des Schaftes ist eine Keilform vorhanden, die dort eine entsprechende Wirkung hervorrufen kann.

Falls zur Vorbereitung des Knochenraums, in welchem der Prothesenschaft eingesetzt soll, eine Raspel oder dergleichen Werkzeug verwendet wird, soll dessen Querschnittsabmessung in seinem dem gerippten Abschnitt des Prothesenschafts entsprechenden Teil nicht größer sein als die des Kerns des Prothesenschafts, damit Knochensubstanz erhalten bleibt, die zwischen der Oberfläche des Schaftkerns und der Innenfläche der harten Knochenrinde komprimiert werden kann.

Der Schaftkern hat mindestens nahe seinem distalen Ende einen rechteckigen Querschnitt. Die an den Längskanten angeordneten Rippen 21 erfüllen infolge ihrer Lage eine besonders starke Führungsfunktion. Selbst wenn irgendein Schaftabschnitt exzentrisch nach ventral oder dorsal versetzt im Markkanal liegen sollte, kann damit gerechnet werden, daß im distalen Abschnitt eine an einer lateralen Kante gelegene Rippe in Eingriff mit der Markraumoberfläche gelangt. Damit dies auch bei besonders ungünstiger Lage des Schafts oder ungünstiger Form des Markraumquerschnitts der Fall ist, sollen nach der Erfindung die an den lateralen Kanten vorgesehenen Rippen auch im Vergleich mit den übrigen lateralen Flächen des Schaftquerschnitts prominent sein, wie dies weiter oben angegeben ist. Entsprechendes gilt für das Verhältnis der lateralen Kantenrippen zu den ventralen bzw. dorsalen Flächenanteilen des Schafts.

## Patentansprüche

1. Hüftprothese mit einem in dem Markkanal des Oberschenkelknochens zu verankernden Schaft (1), dessen distaler, diaphysär zu verankernder Abschnitt (7) einen sich zum Ende hin verjüngenden Kernquerschnitt (12) aufweist, der zumindest auf der lateralen (19) und der medialen Seite (20) Längsrippen (16) aufweist, **dadurch gekennzeichnet, daß** der Kernquerschnitt (12) des Schafts in 1 cm Abstand vom distalen Ende (11) im wesentlichen rechteckig begrenzt ist mit einem Achsenverhältnis von mindestens 1,5, daß er mindestens nahe seinem distalen Ende an seinen beiden lateralen Kanten je eine Rippe (21) trägt und daß die Höhe der Längsrippen von proximal nach distal anwächst.

2. Prothese nach Anspruch 1, **dadurch gekennzeichnet, daß** die Zahl der Rippen (16), die einem auf der lateralen (19) bzw. medialen Seite (20) liegenden Kernbereich zugeordnet sind, mindestens je drei ist.

3. Prothese nach Anspruch 1 oder 2, **dadurch gekennzeichnet, daß** auch auf der Vorder- und Rückseite des distalen Abschnitts Längsrippen (16) vorgesehen sind.

4. Prothese nach einem der Ansprüche 1 bis 3, **dadurch gekennzeichnet, daß** die Verjüngung des Kernquerschnitts über eine Länge von mindestens 4 cm bei durchschnittlich mindestens 8 mm²/cm Länge, vorzugsweise über 10 mm²/cm Länge liegt.

5. Prothese nach einem der Ansprüche 1 bis 4, **dadurch gekennzeichnet, daß** die Verminderung der Querschnittsabmessung in LM-Richtung des distalen Schaftabschnitts (7) über eine Länge von mindestens 4 cm durchschnittlich mindestens 0,5 mm/cm Länge, vorzugsweise mehr als 0,8 mm/cm beträgt.

6. Prothese nach einem der Ansprüche 1 bis 4, **dadurch gekennzeichnet, daß** die Rippenhöhe im Mittel unter 2 mm liegt.

7. Prothese nach Anspruch 6, **dadurch gekennzeichnet, daß** die Rippenhöhe vom proximalen Ende (9) des distalen Abschnitts (7) bis zu dessen distalen Ende (11) von weniger als 0,5 mm auf 0,5 bis 1,5 mm anwächst.

8. Prothese nach einem der Ansprüche 1 bis 7, **dadurch gekennzeichnet, daß** die mittlere Querschnittserstreckung der Rippen in Umfangsrichtung nicht größer als 30 % ihres Mittenabstands ist.

9. Prothese nach einem der Ansprüche 1 bis 8, **dadurch gekennzeichnet, daß** die Flanken der Rippen (16) mit dem Radius durch die Rippenmitte einen Winkel von nicht mehr als 30° einschließen.

10. Prothese nach Anspruch 9, **dadurch gekennzeichnet, daß** die Flanken der Rippen (16) in bezug auf eine Entformungsrichtung (18) nicht hinterschnitten sind.

11. Prothese nach einem der Ansprüche 1 bis 10, **dadurch gekennzeichnet, daß** der Schaftquerschnitt am proximalen Ende (9) des distalen, gerippten Schaftabschnitts (7) oval begrenzt ist mit einem Achsenverhältnis von mindestens 1,2.

12. Prothese nach einem der Ansprüche 1 bis 10, **dadurch gekennzeichnet, daß** der Schaftkernquerschnitt am proximalen Ende (9) im wesentlichen rechteckig begrenzt ist mit einem Achsenverhältnis von mindestens 1,4.

13. Prothese nach einem der Ansprüche 1 bis 12, **dadurch gekennzeichnet, daß** die Begrenzung des Schaftkernquerschnitts zwischen den beiden an den lateralen Kanten befindlichen Rippen (21) nicht weiter lateral vorragt als diese.

14. Prothese nach Anspruch 13, **dadurch gekennzeichnet, daß** eine zwischen den beiden an den lateralen Kanten befindlichen Rippen (21) vorgesehene Rippe nicht um mehr als 2 mm weiter nach lateral vorragt als diese.

15. Prothese nach einem der Ansprüche 1 bis 14, **dadurch gekennzeichnet, daß** auch an den medialen Kanten je eine Rippe vorgesehen ist.

16. Prothese nach Anspruch 15, **dadurch gekennzeichnet, daß** die Begrenzung des Schaftkernquerschnitts zwischen einer an einer lateralen und einer an einer medialen Kante angeordneten Rippe nicht mehr als 1 mm nach ventral bzw. dorsal vorragt.

17. Prothese nach Anspruch 16, **dadurch gekennzeichnet, daß** eine zwischen einer an der lateralen Kante vorgesehenen Rippe (21) und einer an der medialen Kante befindlichen Rippe vorgesehene Rippe nicht um mehr als 2 mm nach ventral bzw. dorsal vorragt als die an den genannten Kanten angeordneten Rippen.

18. Prothese nach einem der Ansprüche 1 bis 17, **dadurch gekennzeichnet, daß** die Rippen rauh ausgebildet sind.

## Claims

1. A hip prosthesis with a shaft (1) to be anchored in the medullary canal of the femur, of which the distal portion (7) to be anchored diaphysially has a core cross-section (12) which tapers towards the end and which at least on the lateral side (19) and on the medial side (20) has longitudinal ribs (16), **characterised in that** the core cross-section (12) of the shaft is bounded substantially rectangularly at a distance of 1 cm from the distal end (11) at an axial ratio of at least 1.5, **in that** at least near its distal end on both of lateral edges it is provided with a respective rib (21), and **in that** the height of the longitudinal ribs increases from the proximal to the distal end.

2. A prosthesis according to Claim 1, **characterised in that** the number of the ribs (16), which are associated with a core portion situated on the lateral side (19) or medial side (20), is at least three.

3. A prosthesis according to Claim 1 or 2, **characterised in that** longitudinal ribs (16) are also provided on the front side and rear side of the distal portion.

4. A prosthesis according to any one of Claims 1 to 3, **characterised in that** the taper of the core cross-section extends over a length of at least 4 cm, with an average of at least 8 mm²/cm length, preferably over 10 mm²/cm length.

5. A prosthesis according to any one of Claims 1 to 4, **characterised in that** the decrease in the cross-sectional dimension in LM direction of the distal shaft portion (7) over a length of at least 4 cm is on average at least 0.5 mm/cm of length, preferably more than 0.8 mm/cm.

6. A prosthesis according to any one of Claims 1 to 4, **characterised in that** the rib height is on average below 2 mm.

7. A prosthesis according to Claim 6, **characterised in that** the rib height from the proximal end (9) of the distal portion (7) to its distal end (11) increases from less than 0.5 mm to 0.5 to 1.5 mm.

8. A prosthesis according to any one of Claims 1 to 7, **characterised in that** the average cross-sectional extension of the ribs in circumferential direction is not greater than 30 % of their centre-to-centre spacing.

9. A prosthesis according to any one of Claims 1 to 8, **characterised in that** the flanks of the ribs (16) form with the radius through the rib centre an angle of not more than 30°.

10. A prosthesis according to Claim 9, **characterised in that** the flanks of the ribs (16) are not undercut in relation to a demoulding direction (18).

11. A prosthesis according to any one of Claims 1 to 10, **characterised in that** the shaft cross-section at the proximal end (9) of the distal ribbed shaft portion (7) is bounded ovally at an axial ratio of at least 1.2.

12. A prosthesis according to any one of Claims 1 to 10, **characterised in that** the shaft cross-section at the proximal end (9) is bounded substantially rectangularly at an axial ratio of at least 1.4.

13. A prosthesis according to any one of Claims 1 to 12, **characterised in that** the boundary of the shaft core cross-section between the two ribs (21) situated at the lateral edges does project laterally further than the latter.

14. A prosthesis according to Claim 13, **characterised in that** a rib provided between the two ribs (21) situated at the lateral edges does not project laterally by more than 2 mm further than the latter.

15. A prosthesis according to any one of Claims 1 to 14, **characterised in that** a respective rib is also provided at the medial edges.

16. A prosthesis according to Claim 15, **characterised in that** the boundary of the shaft core cross-section between a rib disposed on a lateral edge and a rib disposed on a medial edge does not project more than 1 mm ventrally and dorsally respectively.

17. A prosthesis according to Claim 16, **characterised in that** a rib provided between a rib (21) provided at the lateral edge and a rib situated at the medial edge projects ventrally and dorsally respectively by not more than 2 mm than the ribs disposed on said edges.

18. A prosthesis according to any one of Claims 1 to 17, **characterised in that** the ribs are formed rough.

## Revendications

1. Prothèse de la hanche comportant une tige (1) à ancrer dans le canal médullaire de l'os du fémur, dont le tronçon distal (7), à ancrer dans la diaphyse, présente une section du noyau (12) se rétrécissant vers l'extrémité, laquelle comporte des nervures longitudinales (16) au moins sur le côté latéral (19) et le côté médial (20), **caractérisée en ce que** la section du noyau (12) de la tige est délimitée de manière sensiblement rectangulaire à 1 cm de distance de l'extrémité distale (11) avec un rapport des axes d'au moins 1,5, **en ce qu'**au moins à proximité de son extrémité distale, elle porte une nervure (21) sur chacun de ses deux bords latéraux, et **en ce que** la hauteur des nervures longitudinales augmente du côté proximal vers le côté distal.

2. Prothèse selon la revendication 1, **caractérisée en ce que** le nombre de nervures (16), qui sont associées à une zone du noyau se situant sur le côté latéral (19) ou le côté médial (20), est au moins de trois.

3. Prothèse selon la revendication 1 ou 2, **caractérisée en ce que** des nervures longitudinales (16) sont prévues sur le côté avant et le côté arrière du tronçon distal.

4. Prothèse selon l'une des revendications 1 à 3, **caractérisée en ce que** le rétrécissement de la section du noyau sur une longueur d'au moins 4 cm est en moyenne d'au moins 8 mm²/cm de longueur, de préférence supérieur à 10 mm²/cm de longueur.

5. Prothèse selon l'une des revendications 1 à 4, **caractérisée en ce que** la réduction de la dimension de la section dans la direction LM du tronçon distal (7) de la tige, sur une longueur d'au moins 4 cm, est en moyenne d'au moins 0,5 mm/cm de longueur, de préférence supérieure à 0,8 mm/cm.

6. Prothèse selon l'une des revendications 1 à 4, **caractérisée en ce que** la hauteur des nervures est en moyenne inférieure à 2 mm.

7. Prothèse selon la revendication 6, **caractérisée en ce que** la hauteur des nervures depuis l'extrémité proximale (9) du tronçon distal (7) jusqu'à son extrémité distale (11), augmente de moins de 0,5 mm à 0,5 à 1,5 mm.

8. Prothèse selon l'une des revendications 1 à 7, **caractérisée en ce que** l'étendue moyenne de la section des nervures dans la direction périphérique n'est pas supérieure à 30 % de leur distance de centre à centre.

9. Prothèse selon l'une des revendications 1 à 8, **caractérisée en ce que** les flancs des nervures (16) forment, avec le rayon passant par le centre des nervures, un angle non supérieur à 30°.

10. Prothèse selon la revendication 9, **caractérisée en ce que** les flancs des nervures (16) ne sont pas détalonnés par rapport à une direction de démoulage (18).

11. Prothèse selon l'une des revendications 1 à 10, **caractérisée en ce que** la section de la tige à l'extrémité proximale (9) du tronçon distal (7) nervuré de la tige est délimitée par un ovale avec un rapport des axes d'au moins 1,2.

12. Prothèse selon l'une des revendications 1 à 10, **caractérisée en ce que** la section du noyau de la tige à l'extrémité proximale (9) est délimitée sensiblement par un rectangle avec un rapport des axes d'au moins 1,4.

13. Prothèse selon l'une des revendications 1 à 12, **caractérisée en ce que** la délimitation de la section du noyau de la tige entre les deux nervures (21), se trouvant sur les bords latéraux, ne dépasse pas plus loin latéralement que celles-ci.

14. Prothèse selon la revendication 13, **caractérisée en ce qu'**une nervure, prévue entre les deux nervures (21) se trouvant sur les bords latéraux, ne dépasse pas latéralement de plus de 2 mm que celles-ci.

15. Prothèse selon l'une des revendications 1 à 14, **caractérisée en ce qu'**il est prévu aussi une nervure sur chacun des bords médiaux.

16. Prothèse selon la revendication 15, **caractérisée en ce que** la délimitation de la section du noyau de la tige entre une nervure latérale et une nervure disposée sur un bord médial, ne dépasse pas de plus de 1 mm vers le côté ventral ou le côté dorsal.

17. Prothèse selon la revendication 16, **caractérisée en ce qu'**une nervure prévue entre une nervure (21) prévue sur le bord latéral et une nervure se trouvant sur le bord médial, ne dépasse pas de plus de 2 mm vers le côté ventral ou le côté dorsal que les nervures disposées sur lesdits bords.

18. Prothèse selon l'une des revendications 1 à 17, **caractérisée en ce que** les nervures sont réalisées rugueuses.
